# EUROPEAN PATENT APPLICATION

(11) **EP 2 813 207 A1**
(43) Date of publication of application: **17.12.2014**
(21) Application number: 14169608.8
(22) Date of filing: 23.05.2014
(51) Int. Cl.: A61K 8/34, A61Q 19/06, A61K 8/97

(54) **Anti-cellulite product based on natural essences**

(30) Priority: 24.05.2013 ES 201330753
(71) Applicant: Bianco, Roberto, 43892 Rustical Mont Roig - Tarragona (ES)
(72) Inventor: Bianco, Roberto, 43892 Rustical Mont Roig - Tarragona (ES)
(74) Representative: Pons Ariño, Angel

(57) **Abstract**

The present invention relates to a product with anti-cellulite properties whose composition is based on natural essences of vegetable origin, such as thyme, rosemary, bitter orange and spearmint, and at least one cosmetically acceptable vehicle.

## Description

The present invention relates to a product with anti-cellulite properties whose composition is based on natural essences of vegetable origin. Therefore, the invention may be included within the field of cosmetics.

### PRIOR STATE OF THE ART

Cellulite is commonly understood to mean the accumulation of adipose tissue in certain body areas, to form adipose fat nodules. Between 85% and 98% of women after puberty present some degree of cellulite, which is conditioned by genetics and ethnic origin, and is more frequent, for example, in Mediterranean women. In order to prevent its appearance, good nutrition and habitual exercise are recommended, supplemented by topically-applied cosmetic products, especially such as to avoid the unsightly look of the skin in the area with cellulite.

Currently, there are hundreds of products in the market designed to prevent or reduce cellulite, with various degrees of complexity in their composition and more or less costly. These compositions present certain problems: on the one hand, their formulations include chemical products that cause such compounds to exhibit secondary effects in the short or the long term; on the other hand, their results do not reach a desirable level, for which reason none of them combines the four basic, essential actions that are recognised as desirable in the treatment of cellulite, i.e. lipolysis, anti-oedemic properties, circulation activation and regulation of capillary permeability.

### DESCRIPTION OF THE INVENTION

The present invention relates to an anti-cellulite composition that comprises:
- thyme essence,
- rosemary essence,
- bitter orange essence,
- spearmint essence,
- menthol and
- at least one cosmetically acceptable vehicle.

In the present invention, the term "essence" refers to an extract of natural origin, preferably of vegetable origin, formed by various types of molecules, such as alcohols, esters, aldehydes, ketones and lactones and oxides, which are volatile, have a characteristic odour and cause diverse physiological effects. They may be obtained from the flowers, fruits, leaves, roots, seeds and bark of vegetables by methods known to persons skilled in the art, such as distillation or extraction.

The composition of the invention may be presented in any of the pharmaceutical forms known to persons skilled in the art, such as solutions, suspensions, pastes, powders, emulsions, sticks, preparations, foam-forming liquids, gels, aerosols.

In a preferred embodiment, the composition of the invention is presented in the form of a cream, salve or ointment. In this case, a natural gel or an ingredient habitually used to obtain cosmetic products is further added in order to obtain a solid composition.

In another preferred embodiment, the composition of the invention is presented in the form of a lotion. In this case, distilled water or any liquid base used to obtain cosmetics is further added in order to obtain a liquid composition.

In a preferred embodiment, the essences that make up the composition of the invention come from organically-grown plants.

The vehicles other than water that may be used in the compositions according to this invention include solids or liquids, such as emollients, solvents, wetting agents, thickening agents and powders. Examples of each of these types of vehicle, which may be used individually or as mixtures of one or more vehicles, are the following:

Emollients such as stearyl alcohol, glyceryl monoricinoleate, glyceryl monostearate, propane-1,2-diol, butane-1,2-diol, mink oil, cetyl alcohol, isopropyl isostearate, stearic acid, isobutyl palmitate, isocetyl stearate, oleyl alcohol, isopropyl laurate, hexyl laurate, decyl oleate, octadecane-2-ol, isocetyl alcohol, cetyl palmitate, dimethyl polysiloxane, di-n-butyl sebacate, isopropyl myristate, isopropyl palmitate, isopropyl stearate, butyl stearate, polyethylene glycol, triethylene glycol, lanolin, sesame oil, peanut oil, arachis oil, castor oil, acetylated lanolin alcohols, vaseline, mineral oil, butyl myristate, isostearic acid, palmitic acid, isopropyl linoleate, lauryl lactate, myristyl lactate, decyl oleate and myristyl myristate.

Surfactants such as sodium dodecylbenzenesulfonate, abietic acid, tetradecylphosphonic acid dimethyl ether, lauryl monoethanolamide, octylphenol polyethoxylate, glycerol diester (diglyceride), sorbitan esters such as polyethoxysorbitan (Tween 20®), polysorbate 80 (Tween 80®) or sorbitan trioleate 20EO (Tween 85®), dodecyl betaine, n-dodecyl pyridinium chloride.

Propellants such as trichlofluoromethane, dichlorodifluoromethane, dichlorotetrafluoroethane, monochlorodifluoromethane, trichlorotrifluoroethane, propane, butane, isobutane, dimethyl ether, carbon dioxide and nitrous oxide.

Solvents such as ethyl alcohol, methylene chloride, isopropanel, castor oil, ethylene glycol monoethyl ether, diethylene glycol monobutyl ether, diethylene glycol monoethyl ether, dimethylsulfoxide, dimethylformamide and tetrahydrofuran.

Wetting agents such as glycerin, sorbitol, sodium 2-pyrrolidone-5-carboxylate, soluble collagen, dibutyl phthalate and gelatin.

Powders such as chalk, talc, Fuller's earth, kaolin, starch, gums, colloidal silicon dioxide, sodium polyacrylate, tetra-alkyl and/or trialkyl aryl ammonium smectites, chemically-modified magnesium aluminum silicate, organically-modified montmorillonitic clay, hydrated aluminum silicate, pyrolytic silica, carboxyvinyl polymers, sodium carboxymethylcellulose and ethylene glycol monostearate.

Preferably, the quantity of vehicle in the composition, including the water, if present, must be sufficient to transport the natural essences to deepest layers of the skin in a quantity that is sufficient to enhance the effect. The quantity of vehicle may make up the rest of the composition, particularly when the composition has few or no additional ingredients. Therefore, the vehicle or vehicles may make up between 1% and 99.99%, preferably between 50% and 99.5% and, ideally, between 90% and 99% of the composition by weight.

One preferred embodiment of the composition comprises a quantity of thyme essence ranging between 0.2 and 2 ml per kg or litre of total composition.

Another preferred embodiment of the composition comprises a quantity of rosemary essence ranging between 1 and 3 ml per kg or litre of total composition.

Another preferred embodiment of the composition comprises a quantity of bitter orange essence ranging between 0.01 and 1.5 ml per kg or litre of total composition.

Another preferred embodiment of the composition comprises a quantity of spearmint essence ranging between 0.01 and 1.5 ml per kg or litre of total composition.

Another preferred embodiment of the composition comprises a quantity of menthol ranging between 5 and 15 g per kg or litre of total composition.

Another aspect of the invention relates to a process for obtaining the composition described above, which comprises the following steps:
a. mixing the thyme, rosemary, bitter orange and spearmint natural essences,
b. adding the vehicle,
c. adding the menthol and
d. emulsifying the mixture obtained.

Preferably, this process is performed at a temperature ranging between 30°C and 50°C, and, more preferably, at 40°C.

The emulsification step may be performed by means of any physical or chemical technique and device developed for this function and known to any person skilled in the art.

Another aspect relates to a cosmetic method designed for the reduction and/or elimination of cellulite, which comprises the following steps:
a. applying the composition described above on the desired area,
b. covering said area with a transparent plastic for a period of time ranging between 30 and 90 minutes,
c. removing the plastic and the excess composition, and
d. washing the area after step (c).

Preferably, the application of step (a) is performed by means of a glove or directly with a spray.

Throughout the description and the claims, the word "comprises" and the variants thereof are not intended to exclude other technical characteristics, additives, components or steps. For persons skilled in the art, other objects, advantages and characteristics of the invention will arise partly from the description and partly from the practice of the invention. The following example is provided for illustrative purposes, and is not intended to limit the scope of this invention.

### EMBODIMENT EXAMPLE OF THE INVENTION

The anti-cellulite composition of the invention was prepared in the form of a cream, for which 1 kg of natural gel was used as the base; and in the form of a spray lotion, for which 1 litre of distilled water was used as the starting-point. The following ingredients were added to these bases:
2.7 ml of polysorbate 80 PhEur = Tween 80 emulsion
0.5 ml of organic thyme essence
2.4 ml of organic rosemary essence
0.02 ml of organic bitter orange essence
0.06 ml of organic spearmint
10 g of natural menthol in crystal form

The preparation was performed at a temperature of 40°C; first, the essences were mixed; then, the polysorbate and, finally, the menthol in crystal form were added, in order to emulsify all the ingredients in a mixer.

In order to obtain the desired anti-cellulite effect, the product obtained is applied on the area to be treated with a glove or directly with the spray; it is covered with a transparent plastic film and allowed to act for 55 minutes. Once this time has elapsed, the excess is removed with paper and the area is washed once 12 hours have elapsed since the application.

## Claims

1. Anti-cellulite composition that comprises:
• thyme essence,
• rosemary essence,
• bitter orange essence,
• spearmint essence,
• menthol and
• at least one cosmetically acceptable vehicle.

2. Composition according to claim 1, **characterised in that** it is presented in the form of a cream, salve or ointment.

3. Composition according to the preceding claim, which further comprises natural gel.

4. Composition according to claim 1, **characterised in that** it is presented in the form of a lotion.

5. Composition according to the preceding claim, which further comprises distilled water.

6. Composition according to any of the preceding claims, **characterised in that** the essences are obtained from organically-grown plants.

7. Composition according to any of the preceding claims, where the quantity of thyme essence in the total composition ranges between 0.2 and 2 ml per kg or litre of total composition.

8. Composition according to any of the preceding claims, where the quantity of rosemary essence in the total composition ranges between 1 and 3 ml per kg or litre of total composition.

9. Composition according to any of the preceding claims, where the quantity of bitter orange essence in the total composition ranges between 0.01 and 1.5 ml per kg or litre of total composition.

10. Composition according to any of the preceding claims, where the quantity of spearmint essence in the total composition ranges between 0.01 and 1.5 ml per kg or litre of total composition.

11. Composition according to any of the preceding claims, where the quantity of menthol in the total composition ranges between 5 and 15 g per kg or litre of total composition.

12. Process for obtaining the composition according to any of the preceding claims, which comprises the following steps:
a. Mixing the thyme, rosemary, bitter orange and spearmint natural essences,
b. adding the vehicle,
c. adding the menthol and
d. emulsifying the mixture obtained.

13. Process according to the preceding claim, **characterised in that** it is performed at a temperature ranging between 30°C and 50°C.

14. Cosmetic method designed for the reduction and/or elimination of cellulite, which comprises the following steps:
a. applying the composition described according to any of claims 1 to 11 in the selected area,
b. covering said area by means of a transparent plastic for a period of time ranging between 30 and 90 minutes,
c. removing the plastic and the excess composition, and
d. washing the area after step (c).

15. Method according to the preceding claim, where the application of step (a) is performed by means of a glove or directly with a spray
